# EUROPEAN PATENT APPLICATION

(11) **EP 4 831 996 A1**
(43) Date of publication of application: **28.10.2026**
(21) Application number: 25305582.6
(22) Date of filing: 23.04.2025
(51) Int. Cl.: G16H 10/20, G16H 20/70

(54) **DEVICE FOR AUTOMATIC ADAPTATION OVER TIME OF AN ADAPTATIVE THERAPY FOR A USER AFFECTED BY A MENTAL DISORDER**

(71) Applicant: Emobot, 75001 Paris (FR)
(72) Inventor: PETELOT, Tanel, 75010 PARIS (FR); PERZO-MESSIAS, Antony, 75005 PARIS (FR)
(74) Representative: Icosa

(57) **Abstract**

The present invention relates to a device and associated computer-implemented method for automatic adaptation over time of an adaptative therapy for a user affected by a mental disorder, wherein said adaptative therapy is previously prescribed by a healthcare professional and to a biofeedback device and associated computer-implemented method for self-adaptation of an adaptative therapy previously prescribed by a healthcare professional to a user affected by a mental disorder.

## Description

### FIELD OF INVENTION

The present invention relates to the field of automatic adaptation of personalized digital therapy. In particular, the present invention relates to a device and associated computer-implemented method for automatic adaptation over time of an adaptative therapy for a user affected by a mental disorder, and to a device and associated computer-implemented method for self-adaptation of an adaptative therapy previously prescribed by a healthcare professional to a user affected by a mental disorder.

### BACKGROUND OF INVENTION

Existing solutions for adapting digital therapies (DTx) to users with mental disorders (e.g. mood disorders) face several critical limitations. Many current approaches rely on automated mood tracking to personalize therapy, but they often use complex, non-transparent algorithms that make it difficult for healthcare professionals to understand and oversee the decision-making process. This lack of explainability can lead to a loss of trust in the system and hesitation to integrate such technologies into clinical practice. Instead, therapy personalization is often based on predefined protocols or user-selected preferences, which may not accurately reflect treatment needs.

Some systems rely on fixed schedules, random selection, or self-reported mood assessments for adjusting therapeutic exercises over time, which can be subjective and burdensome for users. These approaches may not provide a reliable or continuous measure of progress, making it difficult to determine whether the therapeutic exercise is effectively addressing symptoms. Furthermore, users undergoing a digital therapy without supervision often struggle with motivation, engagement, and adherence, leading to high dropout rates. The absence of an objective feedback mechanism to track progress and validate improvements exacerbates this issue, as users may feel uncertain about their progress or disconnected from the therapeutic process.

The present invention aims to address such limitations.

### SUMMARY

This invention thus relates to a device for automatic adaptation over time of an adaptative therapy for a user affected by a mental disorder, wherein said adaptative therapy is previously prescribed by a healthcare professional, said device comprising:
- at least one input configured to receive:
   ∘ at least one user data relative from the user, said at least one user data being associated with at least one symptom of said mental disorder;
   ∘ a set of C therapy categories, wherein each category:
      ▪ comprises a group of therapeutic exercises related to said mental disorder, and
      ▪ is associated, for each of said at least one symptom, with a predefined relation score indicative of the relatedness of said therapy category to said symptom;
- at least one processor configured to automatically adapt said adaptative therapy for the user by iteratively obtaining, for each current iteration, an adapted current therapy to be proposed to the user to improve the user current mental state by:
   ∘ obtaining the at least one user data from at least one time period anterior to the current iteration;
   ∘ ranking said therapeutic exercises based on said associated predefined relation scores and said at least one user data so as to obtain a ranked set of therapeutic exercises;
   ∘ generating the adapted current therapy based on said ranked set of therapeutic exercises, so that at least one therapeutic exercise comprised in the highest ranked therapeutic exercises is included in the adapted current therapy;
- at least one output configured to provide said adapted current therapy as generated at each iteration, to be proposed to the user to improve the user current mental state.

Advantageously, the device for automatic adaptation over time of an adaptative therapy allows for a personalized digital therapy (DTx), where the most suitable therapeutic exercises are suggested in the adapted current therapy based on the user's health status. This solution enhances user engagement by actively involving them in their treatment while reducing the cognitive burden of choosing therapeutic exercises from an overwhelming selection. Furthermore, the device allows a user-centered therapy adaptation for improved therapeutic effectiveness and user experience. Indeed, the device for automatic adaptation over time of an adaptative therapy through a seamless interaction between automated monitoring and user-centered adaptation allowing to optimize therapeutic effectiveness and user experience. Another advantage of this invention is that it offers an explainable and transparent solution for users and healthcare professionals, where parameters are clearly defined and easy to understand, ensuring better decision-making.

According to other advantageous aspects of the invention, the device comprises one or more of the features described in the following embodiments, taken alone or in any possible combination.

According to an embodiment, the device is further configured to receive as input:
- a history of the therapeutic exercises, comprising a collection of therapeutic exercises previously performed by the user and an associated biofeedback for each therapeutic exercise previously performed;
- at least one biofeedback of the user, each of said at least one biofeedback relating to the effect of one therapeutic exercise;
wherein said at least one processor is further configured to:
- update the history of the therapeutic exercises using the latest at least one biofeedback of the user;
- for each therapeutic exercise of each therapy category, estimate a user exercise weight being representative of an effect of said therapeutic exercise using said updated history of the therapeutic exercises,
- obtain a priority score for each therapeutic exercise in each therapy category using said at least one user exercise weight, said at least one user data and said predefined relation scores; and wherein the ranking of the therapeutic exercises is performed using said priority scores and wherein the at least n higher ranked therapeutic exercises are added to the adapted current therapy.

According to an embodiment, said at least one user data relative to the user is one of the following:
- a health score obtained from a questionnaire;
- a biometric data obtained from a sensor associated to the user;
- a temporal variation of a severity of at least one symptom related to said mental disorder.

According to an embodiment, each category of said set of C therapy categories is one of the following:
- learning a behavior, such as assertiveness;
- learning awareness of maladaptive and unrealistic thoughts;
- learning to accept various emotions;
- learning to relax physically and psychologically;
- gradual exposure to a stressful situation, such as phobia;
- increasingly frequent exposure to rewarding situations;
- mindfulness exercises;
- Progressive Muscle Relaxation (PMR) exercices;
- developing human relations through role-playing or a game;
- Dialectical Behavior Therapy (DBT) exercises;
- Acceptance and Commitment Therapy (ACT) exercises;
- Cognitive Behavioral Therapy (CBT) exercises;
- Rational Emotive Behavior Therapy (REBT) exercises;
- Positivist Psychology exercices.

According to an embodiment, the device is further configured to receive as input a preference of said user, and wherein said ranking of said therapeutic exercises is further based on said preference of said user.

According to an embodiment, before ranking said therapeutic exercises, said set of C therapy categories is filtered according to a received preference of said healthcare professional. Advantageously, this allows for a medical oversight over the adaptative therapy ensuring an optimized therapeutic effectiveness.

According to an embodiment, said at least one user data is obtained according to a predefined schedule from the user so as to update said current therapy on said predefined time interval.

According to an embodiment, said predefined relation score is reported from a relation data structure comprising predefined relation scores for each symptom associated to said mental disorder and each therapy category, so as to associated a predefined relation score to a pair symptom - therapy category.

According to an embodiment, information relating to said adapted current therapy is provided to the user through an interface, and said ranked therapeutic exercises are presented to the user in a descending rank order by: presenting first a therapeutic exercise with the highest rank and last a therapeutic exercise with the lowest rank.

According to an embodiment, when two therapeutic exercises have an equal rank, the therapeutic exercise associated with the longest time interval since its previous use is presented before the therapeutic exercise associated with a shorter time interval since its previous use.

According to an embodiment, wherein in the current therapy under administration is updated by removing a therapeutic exercise once said user uses said therapeutic exercise.

According to an embodiment, a predefined time interval elapses between two consecutive iterations.

This invention further relates to a biofeedback device for self-adaptation of an adaptative therapy previously prescribed by a healthcare professional to a user affected by a mental disorder, said biofeedback device comprising:
- at least one input configured to receive:
   o an adapted current therapy obtained by a device according to any one of the previous embodiments of the device for automatic adaptation over time of an adaptative therapy;
   ∘ at least one previous user data relative to the user, said at least one previous user data being associated with at least one symptom of said mental disorder previous to the prescription of said adapted current therapy;
- at least one processor configured to:
   o provide said adapted current therapy to said user;
   o collect at least one current user data from said user, said at least one current user data being associated with at least one symptom of said mental disorder after the user had performed at least one of the therapeutic exercises comprised in the adapted current therapy;
   ∘ compare said at least one current user data to said at least previous user data so as to obtain a user health score;
   ∘ compare said user health score to a predefined user health threshold;
   ∘ output to the user said user health score to provide a biofeedback on improvement of their current mental state;
   ∘ repeat: collect at least one current user data from said user, compare said at least one current user data, and compare said user health score, until the user health score reaches or exceeds the predefined user health threshold associated to a user health status.

Advantageously, the device for self-adaptation of a therapy enables continuous monitoring of the user's health status, ensuring precise tracking of the evolution of the user health status over time. This personalized approach optimizes therapeutic effectiveness. Additionally, the device for self-adaptation of a therapy provides the user with direct insight into their progress through a comprehensive scoring system (using the user health score), allowing them to visualize improvements (e.g. through their mobile or tablet) and stay actively engaged in their therapeutic journey. Indeed, the device for self-adaptation of a therapy allows a virtuous loop-like adaptation of the therapy based on the user's follow up data (e.g. previous user data, current user data) making sure that the user's health status evolves towards a healthier state.

The disclosure further relates to a computer-implemented method for automatic adaptation over time of an adaptative therapy for a user affected by a mental disorder, wherein said adaptative therapy is previously prescribed by a healthcare professional, said method comprising:
- receiving:
   ∘ at least one user data relative to the user, said at least one user data being associated with at least one symptom of said mental disorder;
   ∘ a set of C therapy categories, wherein each category:
      ▪ comprises a group of therapeutic exercises related to said mental disorder, and
      ▪ is associated, for each of said at least one symptom, with a predefined relation score indicative of the relatedness of said therapy category to said symptom;
- automatically adapting said adaptative therapy for the user by iteratively obtaining, for each current iteration, an adapted current therapy to be proposed to the user to improve the user current mental state by:
   ∘ obtaining the at least one user data from at least one time period anterior to the current iteration;
   ∘ ranking said therapeutic exercises based on said associated predefined relation scores and said at least one user data so as to obtain a ranked set of therapeutic exercises;
   ∘ generating the adapted current therapy based on said ranked set of therapeutic exercises, so that at least one therapeutic exercise comprised in the highest ranked therapeutic exercises is included in the adapted current therapy;
- providing said adapted current therapy as generated at each iteration, to be proposed to the user to improve the user current mental state.

The disclosure further relates to a computer-implemented method for self-adaptation of an adaptative therapy previously prescribed by a healthcare professional to a user affected by a mental disorder, said method comprising:
- receiving:
   ∘ an adapted current therapy obtained by a method/device for automatic adaptation over time of an adaptative therapy according to any embodiment of the present disclosure;
   ∘ at least one previous user data relative to the user, said at least one previous user data being associated with at least one symptom of said mental disorder previous to the prescription of said adapted current therapy;
- providing said adapted current therapy to said user;
- collecting at least one current user data from said user, said at least one current user data being associated with at least one symptom of said mental disorder after the user had performed at least one of the therapeutic exercises comprised in the adapted current therapy;
- comparing said at least one current user data to said at least previous user data so as to obtain a user health score;
- comparing said user health score to a predefined user health threshold;
- outputting to the user said user health score to provide a biofeedback on improvement of their current mental state;
- repeating: collect at least one current user data from said user, compare said at least one current user data, and compare said user health score, until the user health score reaches or exceeds the predefined user health threshold associated to a user health status.

In addition, the disclosure relates to a computer program comprising software code adapted to perform a method for automatic adaptation over time of an adaptative therapy and/or a method for self-adaptation of an adaptative therapy compliant with any of the above execution modes when the program is executed by a processor.

The present disclosure further pertains to a non-transitory program storage device, readable by a computer, tangibly embodying a program of instructions executable by the computer to perform a method for automatic adaptation over time of an adaptative therapy and/or a method for self-adaptation of an adaptative therapy, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:
The terms "adapted" and "configured" are used in the present disclosure as broadly encompassing initial configuration, later adaptation or complementation of the present device, or any combination thereof alike, whether effected through material and/or software means (including firmware).

The term "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:
**Figure 1** is a block diagram representing schematically a particular mode of a device for automatic adaptation over time of an adaptative therapy compliant with the present disclosure;
**Figure 2** is a block diagram representing schematically a particular mode of a device for self-adaptation of an adaptative therapy compliant with the present disclosure;
**Figure 3** is a flow chart showing successive steps executed with the device for automatic adaptation over time of an adaptative therapy of figure 1;
**Figure 4** is a flow chart showing successive steps executed with the device for self-adaptation of an adaptative therapy of figure 2;
**Figure 5** diagrammatically shows an apparatus integrating the functions of the device for automatic adaptation over time of an adaptative therapy of figure 1 and of the device for self-adaptation of a therapy of figure 2;
**Figure 6** is an example of patient follow-up journey;
**Figure 7** is an example of optimizing the digital therapy;
**Figure 8** is an example of fine-tuning at exercise level.

On the figures, the drawings are not to scale, and identical or similar elements are designated by the same references.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

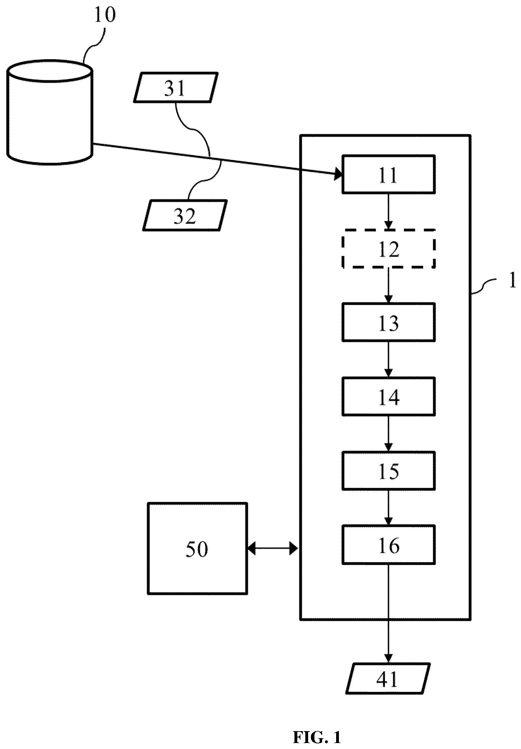

The present disclosure will be described in reference to a particular functional embodiment of a device 1 for automatic adaptation over time of an adaptative therapy (i.e. digital therapy) for a user affected by a mental disorder, as illustrated on **Figure 1****.**

For example, the mental disorder may be depressive disorder, anxiety disorder, bipolar disorder, post-traumatic stress disorder (PTSD), schizophrenia, eating disorder, disruptive dissocial behavior, neurodevelopmental disorder, Parkinson's disease, Alzheimer's disease, or any type of mood disorders.

Device 1 is adapted to produce (to output) an adapted current therapy 41 as generated at each iteration, to be proposed to the user to improve the user current mental state.

Device 1 comprises at least one input configured to receive:
- at least one user data 31 relative to the user, the at least one user data being associated with at least one symptom of the mental disorder; and
- a set of C therapy categories 32, each category comprising a group of therapeutic exercises related to the mental disorder, and each therapy category is associated, for each of the at least one symptom, with a predefined relation score indicative of the relatedness of the therapy category to the symptom.

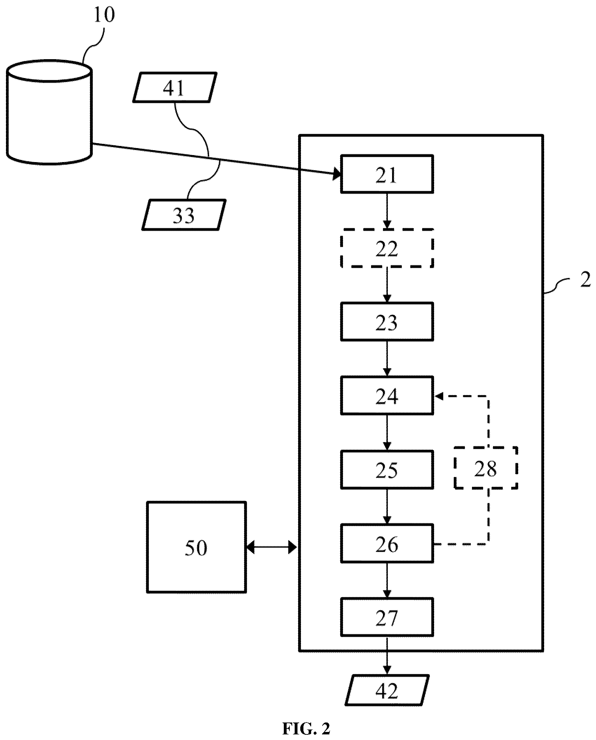

In some examples, the device 1 for automatic adaptation over time of an adaptative therapy may be associated with a biofeedback device 2 for self-adaptation of an adaptative therapy, represented on **Figure 2****,** which will be subsequently described.

Though the presently described devices 1 and 2 are versatile and provided with several functions that can be carried out alternatively or in any cumulative way, other implementations within the scope of the present disclosure include devices having only parts of the present functionalities.

Each of the devices 1 and 2 is advantageously an apparatus, or a physical part of an apparatus, designed, configured and/or adapted for performing the mentioned functions and produce the mentioned effects or results. In alternative implementations, any of the device 1 and the biofeedback device 2 is embodied as a set of apparatus or physical parts of apparatus, whether grouped in a same machine or in different, possibly remote, machines. Device 1 and/or biofeedback device 2 may e.g. have functions distributed over a cloud infrastructure and be available to users as a cloud-based service, or have remote functions accessible through an API.

Device 1 for automatic adaptation over time of an adaptative therapy and biofeedback device 2 for self-adaptation of a therapy may be integrated in a same apparatus or in set of apparatus, and intended to same users. In other implementations, the structure of biofeedback device 2 may be completely independent of the structure of device 1, and may be provided for other users. Device 1 and/or biofeedback device 2 may for example be a smartphone or a tablet or a personal computer.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules may be implemented in hardware, software, firmware, or any mixed form thereof as well. They are preferably embodied within at least one processor of device 1 or of biofeedback device 2.

Device 1 comprises a module 11 for receiving the at least one user data 31 obtained directly or indirectly from the user and the set of C therapy categories 32 with their associated therapeutic exercises, symptoms and predefined relation scores. Module 11 constitutes the at least one input configured to receive such data. These data may for example have been stored in one or more local or remote database(s) 10, which may or may not be part of device 1. The database(s) can take the form of storage resources available from any kind of appropriate storage means, which can be notably a RAM or an EEPROM (Electrically-Erasable Programmable Read-Only Memory) such as a Flash memory, a HDD (Hard Disk Drive), or an SSD (Solid-State Disk).

The at least one user data 31 may for example be:
- a health score obtained from a questionnaire (e.g. a health score previously obtained from a questionnaire answered by the user), such as for example a mood-related questionnaire (e.g. PHQ-9, MADRS, HAD);
- a biometric data obtained from a sensor associated to the user such as heart rate variability measured via a wearable sensor to assess stress and emotional status, facial expression captured through a camera to detect mood changes, voice tone modulation analyzed via a microphone to monitor emotional fluctuations;
- a temporal variation (e.g. temporal derivative) of a severity of at least one symptom related to the mental disorder (mental disorder affecting the user).

It is to be understood that these user data have, at some point in time before use of the device 1, been stored in the said database in relation to said user (e.g. associated to a user identifier). For example, the healthcare professional may prescribe the solution (computer-implemented method 100 and/or computer-implemented method 200) to a user by logging into a web page and generating a user identifier that may be sent to the user (e.g. to the user's email address or mobile phone). This user identifier may be used by the user when installing the solution on their smartphone and/or laptop. During the installation of the solution, the user may follow a procedure to locally register a picture of their face for instance (on the smartphone or laptop). This registration may be later used to identify the user and analyze their behavior, in case they are correctly identified.

Each category of the set of C therapy categories may for example comprise Cognitive Behavioral Therapy (CBT) therapy exercises.

A therapy category may for example be a "Sources of self-esteem" category comprising therapeutic exercises such as for example a therapeutic exercise for "Moderating the inner critic" that may suggest writing negative criticisms such as for example "I lack willpower" and for each negative criticism: (i) identifying whether it is a criticism based on facts or hypotheses, (ii)reformulating the criticism in a positive form such as "I have willpower, the proof, on such and such an occasion, I demonstrated willpower", and (iii)each week, force yourself to identify facts proving that I have willpower "I have managed to only drink alcohol on weekends for a month".

"Sources of self-esteem" category may further comprise a therapeutic exercise such as a "Getting stronger" exercise that may comprise for the user writing down on a sheet of paper different areas such as family, work, hobbies, etc. and for each of these areas, firstly, listing their strengths and then identifying the activities the user would like to develop. For example, "I don't spend much time on climbing, which I enjoyed when I was twenty, so I decided to look for a climbing club in my neighborhood.".

A therapy category may for example be a "Social relations" category comprising therapeutic exercises such as for example:
- "Make the effort to reach out to others": Once a week, initiate a personal interaction with the exercise of taking the time to listen to others and try to understand them; this may be done with work colleagues, in the family setting or when walking your pet.
- "Associative environment": Set yourself the goal of joining an association and put together an action plan to this end: 1/ list five activities that bring you well-being; 2/ for each of them, identify the existing associations in your area, 3/ meet them to find out more about joining."

A therapy category may for example be a "Dynamism" category comprising therapeutic exercises such as for example: Play sports, Force yourself to walk fast, Reduce alcohol consumption.

Each therapeutic exercise may for example take the form of an explanatory document. For example, a therapeutic exercise "Smile" would involve asking the user to read an associated document explaining the benefits of smiling. The document could explain what neurofeedback is and how our perception of context changes when we try to smile.

Each therapy category may be associated for each symptom with a predefined relation score indicative of the relatedness of the therapy category to the symptom, in other words the predefined relation score comprises the global effect on the symptom of all the therapeutic exercises comprised in the therapy category. In an example, one predefined relation score is associated for each pair of symptom-therapy category. A predefined relation score related to a symptom may for example be a score indicative of how much therapeutic exercises of the therapy category may positively act on the symptom. For instance, if the therapeutic exercises of a therapy category are the most efficient on a given symptom, the predefined relation score associated to this given symptom may be the highest score among all the predefined relation scores for all the therapy categories. A predefined relation score may for example be fixed by a healthcare professional, and/or obtained from existing clinical studies. Advantageously, therapy categories and their associated predefined relation scores allow to obtain the priority scores of therapeutic exercises which are not accessible at the symptom level due to a lack of granularity in available clinical data. In other words, because the direct relationship between therapeutic exercises and symptoms cannot be assessed from available clinical data, the priority scores are usually unknown without the present invention. By leveraging therapy categories association with symptoms, this lack of information at the level of therapeutic exercises can be overcome.

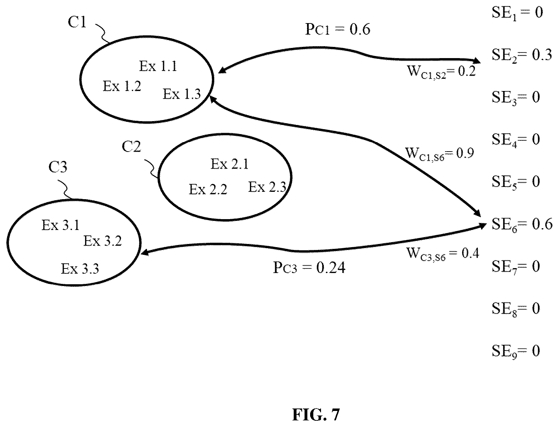

The predefined relation score may be reported from a relation data structure comprising predefined relation scores for each symptom associated to the mental disorder and each therapy category, so as to associate a predefined relation score to a pair symptom - therapy category. Such a relation data structure may be a matrix comprising a predefined relation score for each group of symptom - therapy category, as illustrated hereunder with an example (table 2). For instance, therapy category 1 may comprise three exercises Ex 1.1, Ex 1.2, Ex 1.3, therapy category 2 may comprise two exercises Ex 2.1, Ex 2.2, and therapy category 3 may comprise four exercises Ex 3.1, Ex 3.2, Ex 3.3, Ex 3.4; for each therapy category, a different value of a priority score may be associated to each therapeutic exercise depending on the severity of the symptom and the relation score as displayed in Figure 7. Each therapeutic exercise belonging to the same therapy category may have the same priority score at initialization. The priority score for each therapeutic exercise may change with time during adaptation of the adaptative therapy while the predefined relation scores stay constant. A higher value of a relation score may indicate that a therapy category may strongly improve a symptom, while a lower value of a relation score may indicate that the therapy category is less likely to improve this symptom.

**Table 1: Example table representing the relationship between therapy categories, therapeutic exercises, symptoms and relation scores**

| **Therapy category** | **Exercises** | **Symptoms** | **Relation scores (** ${W}_{Ci , Si , i ∈ ℕ} *$**)** |
|---|---|---|---|
| **Category 1** | Ex 1.1 (C1,S1) | S1 | 9 |
| | Ex 1.2 (C1,S3) | S3 | 2 |
| | Ex 1.3 (C1,S5) | S5 | 5 |
| **Category 2** | Ex 2.1 (C2,S2) | S2 | 4 |
| | Ex 2.2 (C2,S3) | S3 | 1 |
| **Category 3** | Ex 3.1 (C3,S1) | S1 | 8 |
| | Ex 3.2 (C3,S2) | S2 | 3 |
| | Ex 3.3 (C3,S6) | S6 | 7 |
| | Ex 3.4 (C3,S12) | S12 | 3 |

Here's an example of a relation data structure comprising predefined relation scores indicative of the relatedness of n therapy categories to m symptoms (n and m ∈ N*).

**Table 2: Example of relation data structure**

| | **Symptom 1 (S1)** | **Symptom 2 (S2)** | **...** | **Symptom m (Sm)** |
|---|---|---|---|---|
| **Therapy Category 1 (C1)** | **Relation** score | **Relation** score | ... | **Relation** score |
| | W_{C1,S1} | W_{C1,S2} | | W_{C1,Sm} |
| **Therapy Category 2 (C2)** | **Relation** score | Relation score | ... | Relation score |
| | W_{C2,S1} | W_{C2,S2} | | W_{C2,Sm} |
| **...** | ... | ... | ... | ... |
| **Therapy Category n (Cn)** | Relation score | Relation score | ... | Relation score |
| | W_{Cn,S1} | W_{Cn,S2} | | W_{Cn,Sm} |

In some examples, module 11 may further receive:
- a history of the therapeutic exercises, comprising a collection of therapeutic exercises previously performed by the user and optionally an associated biofeedback for each therapeutic exercise previously performed; and/or
- at least one biofeedback of the user relating to the effect of one therapeutic exercise.

The history of the therapeutic exercises may comprise a number of exercises performed by the user in the past; each of these therapeutic exercises may in some examples be associated with a biofeedback that may be for example:
- user respiratory rate (e.g. acquired using a breath sensor), for instance when the therapeutic exercise involves a breathing exercise;
- user heart rate (e.g. acquired using a heart rate sensor), for instance when the therapeutic exercise involves stress management ;
- user body temperature (e.g. using a wearable sensor that tracks peripheral skin temperature), for instance when the therapeutic exercise involves reducing muscle tension and inducing relaxation ;
- user oxygen saturation (e.g. measured via a fingertip sensor, or a sensor on the user's mobile phone) for instance when the therapeutic exercise involves calming the nervous system and improving focus.

The at least one biofeedback of the user relating to the effect of one therapeutic exercise may correspond to a biofeedback collected from the user just before the start of the automatic adaptation over time of the adaptative therapy for the user so as to update the history of the therapeutic exercises. Such a biofeedback may be heart rate, muscle activity, temperature, respiratory rate, blood pressure, oxygen saturation, and may be obtained using a monitoring device associated to the user. The monitoring device may collect audio data, video data, physiological data (e.g., heart rate, skin conductance), motion data (e.g., body movements, posture), and textual data (e.g., speech transcripts, written inputs) or any other type of data related to a symptom of the mental disorder.

Device 1 further comprises optionally a module 12 for preprocessing received inputs such as the at least one user data 31 relative to the user and the set of C therapy categories 32 with their associated symptoms and predefined relation scores. Module 12 may notably be adapted to standardize the received at least one user data 31 for sake of efficient and reliable processing. Such a standardization may be particularly useful when receiving several user data 31 originating from different sources, including possibly different sensors. Thanks to standardization, differences between sources may then be neutralized or minimized. This may make the device 1 more efficient and reliable. In an embodiment, module 12 may also filter the set of C therapy categories according to a received preference of the healthcare professional. For instance, the healthcare professional may have access to the C therapy categories through a medical dashboard through which he/she can choose a particular therapy category or more of them in accordance with the mental disorder of the user. The healthcare professional may give a preference to at least one symptom of the mental disorder to prioritize in the automatic adaptation over time of the adaptative therapy; the preference of the healthcare professional may be received by device 1 which may filter the set of C therapy categories taking into account this received preference. Advantageously, this ensures that the healthcare professional remains actively involved in therapeutic decisions.

Filtering the set of C therapy categories may comprise selecting one or more therapy categories based on symptom(s) of the user to prioritize for the mental disorder of the user; this may be done using a relating data structure comprising associations of pairs of symptom - therapy category.

According to various exemplary configurations, module 12 may be adapted to execute only part or all of the above functions, in any possible combination, in any manner suited to the following processing stage.

Device 1 further comprises at least one processor configured to automatically adapt the adaptative therapy for the user by iteratively obtaining, for each current iteration, an adapted current therapy to be proposed to the user to improve the user current mental state using at least modules 13 (optionally modules 13a, 13b, 13c), 14, and 15.

Device 1 may further comprise a module 13 for obtaining the at least one user data from at least one time period anterior to the current iteration. For example, the at least one time period anterior to the current iteration may be 1 hour, 1 day, 7 days or 15 days prior to the date and/or time at the current iteration.

Device 1 may further comprise an optional module 13a for updating the history of the therapeutic exercises using the latest at least one biofeedback of the user. For example, if the biofeedback indicates an exacerbation of a symptom, the therapeutic exercise performed by the user will be negatively associated with that symptom, meaning it will not be recommended later for improving this symptom. For instance, as illustrated in the example of Figure 7, a therapeutic exercise of the therapy category 1 (C1) is supposed to reduce the severity of the symptom S2 (relation score W_{C1,S2} of 0.2); if the at least one biofeedback of the user indicates an exacerbation of symptom S2 after the user has used the therapeutic exercise of therapy category C1, the relation score may be set to 0 so as this therapeutic exercise would not be taken into consideration in obtaining the priority score of this therapeutic exercise. At initialization, the priority scores may be the same for all therapeutic exercises within a given therapy category. For example, the priority scores for all therapeutic exercises within a given therapy category may be equal to the predefined relation scores indicative of the relatedness of said given therapy category to the different symptoms. In an example, the priority score for a therapy category may be equal to the sum of predefined relation scores multiplied by the severity of the symptoms as presented in example 3. However, as device 1 adapts the adaptative therapy over time, the priority scores of two therapeutic exercises from the same therapy category may become different.

Device 1 may further comprise an optional module 13b for estimating a user exercise weight being representative of an effect of the therapeutic exercise using the updated history of the therapeutic exercises, and that for each therapeutic exercise of each therapy category. For example, in an anxiety management therapy, if a user performs a mindfulness exercise, the monitoring module could track changes in heart rate and stress levels before and after the exercise. By analyzing this data, module 13b may estimate the therapeutic effect of the mindfulness exercise, such as how much the heart rate decreases or how anxiety levels are reduced over time, indicating the effectiveness of this therapeutic exercise.

Device 1 may further comprise an optional module 13c for obtaining a priority score for each therapeutic exercise in each therapy category using the at least one user exercise weight (e.g. past effectiveness of the therapeutic exercise, preference of the user for the therapeutic exercise), the at least one user data (e.g. symptom severity variation for the category) and the predefined relation scores (e.g. severity of symptoms targeted by the category, medical priority assigned to the category). For each therapeutic exercise of a category, a priority score may be obtained as a weighted sum of: a weighted average of symptom severity variation for the category, a weighted average severity of symptoms targeted by the category, a medical priority assigned to the category (i.e. preference of the healthcare professional), a past effectiveness of the therapeutic exercise (i.e. a biofeedback associated with the therapeutic exercise previously performed), a preference of the user for the therapeutic exercise. Advantageously, this allows to obtain for each therapeutic exercise a priority score based on both clinical and personal considerations.

In an example, if a therapeutic exercise has a positive effect on a symptom, its priority score may be reinforced; however, if it has a negative effect, the priority score may be reduced.

Device 1 may further comprise a module 14 for ranking the therapeutic exercises based on the associated predefined relation scores and the at least one user data, as received by module 11 and obtained by module 13, so as to obtain a ranked set of therapeutic exercises. Ranking the therapeutic exercises may comprise mapping therapeutic exercises to the relevant symptoms, with higher-ranked therapeutic exercises being more effective in addressing the user's specific symptoms. For a particular mental disorder, mapping of therapeutic exercises to relevant symptoms may be achieved based on information comprised in any available guideline in the field. Such guidelines may include, for example, the "Affections psychiatriques de longue durée - Troubles anxieux graves" published by the "Haute Autorité de Santé" (Saint-Denis La Plaine, France, 2007), or the World Health Organization (WHO) guidelines related to anxiety disorders. A person skilled in the art will understand that these references are provided for illustrative purposes only and are not limiting. Any other similar guideline in the field of psychology or psychiatry may also be used.

For example, the higher the priority score of a therapeutic exercise, the more the exercise is considered to be associated with a relevant symptom. Predefined relation scores may allow us to evaluate for each symptom which are the most relevant therapeutic exercises (group of therapeutic exercises within a therapy category). For example, therapeutic exercises Ex 1.2 and Ex 2.2 as presented previously may allow to slightly improve symptom S3. For instance, if a user experiences anxiety, therapeutic exercises related to reducing anxiety symptoms will receive higher scores, while therapeutic exercises targeting symptoms of depression may receive lower scores, as they are less relevant to the user's current health status. Advantageously, this ensures that therapeutic exercises are tailored to treat the user's symptoms most effectively.

In an embodiment, the ranking of the therapeutic exercises is performed using the priority scores and the at least n higher ranked therapeutic exercises are added to the adapted current therapy. For instance, the therapeutic exercises may be ranked by ranking first the therapeutic exercise with the highest priority score, second the therapeutic exercise with the second highest priority score, etc. and last the therapeutic exercise with the lowest priority score. Among the therapeutic exercises, an integer number (n) of exercises may be selected to be added to the adapted current therapy.

In an embodiment, device 1 may be further configured to receive as input (notably though module 11) a preference of the user, and the ranking of the therapeutic exercises may be further based on the preference of the user. For instance, if a user does not like a specific therapeutic exercise, the therapeutic exercise may be ranked last or even removed from the group of therapeutic exercises. A preference of the user may comprise time spent on a therapeutic exercise, or an indicator of completion of a therapeutic exercise.

In an embodiment, information relating to said adapted current therapy may be provided to the user through an interface (e.g. mobile application), and the ranked therapeutic exercises may be presented to the user in a descending rank order by: presenting first a therapeutic exercise with the highest rank and last a therapeutic exercise with the lowest rank. For instance, the user may start with the first therapeutic exercise, which is likely the most effective for addressing their specific symptom and the one that aligns with their preferences. The user may repeat this first exercise several times or choose to move on to the second exercise. This way, the user can easily see the most recommended therapeutic exercises first, followed by the less prioritized ones. This flexibility allows the user to adjust their therapy according to their needs and preferences.

In an embodiment, when two therapeutic exercises have an equal rank, the therapeutic exercise associated with the longest time interval since its previous use is presented before the therapeutic exercise associated with a shorter time interval since its previous use. In other words, the therapeutic exercise that that has the most time elapsed since the user last used it may be presented first.

Device 1 may further comprise a module 15 for generating the adapted current therapy based on the ranked set of therapeutic exercises, so that at least one therapeutic exercise comprised in the highest ranked therapeutic exercises is included in the adapted current therapy. In an example, the complete set of therapeutic exercises may be included in the adapted current therapy. In another example, at least a predefined number of therapeutic exercises which have the highest ranks is included in the adapted current therapy.

A predefined time interval (e.g. 1 day, 2 days, 1 week, 2 weeks, 1 month) may elapse between two consecutive iterations.

In some examples, the at least one user data may be obtained from the user according to a predefined schedule so as to update the current therapy on the predefined time interval. In an embodiment, the predefined schedule may correspond to the predefined time interval between two consecutive iterations; for example, the at least one user data may be obtained every 24 hours and 24 hours may elapse between two consecutive iterations.

Device 1 may further comprise a module 16 for providing (outputting) the adapted current therapy 41 as generated at each iteration, to be proposed to the user to improve the user current mental state. In an example, the adapted current therapy 41 may be provided to a healthcare professional through a medical dashboard for medical validation. For example, at each current iteration, the medical dashboard may be updated presenting to the healthcare professional the adapted current therapy 41, and other user data such as at least one user data 31 relative to the user and/or at least one user data from at least one time period anterior to the current iteration.

In an embodiment, the current therapy under administration may be updated by removing a therapeutic exercise from the adapted current therapy 41 once the user uses or has used the therapeutic exercise.

It may be observed that the operations performed by the modules 11, 12, 13, 13a, 13b, 13c, 14, 15, 16, and 17 are not necessarily successive in time, and may overlap, proceed in parallel or alternate, in any appropriate manner.

In some examples, the device 1 is interacting with a user interface 50, via which information can be entered and retrieved by the user. The user interface 50 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

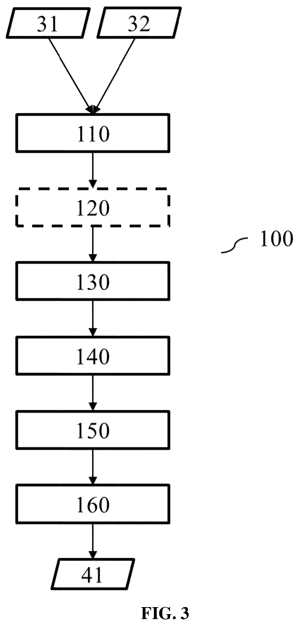

In its automatic actions, the device 1 may for example execute the following computer-implemented method 100 for automatic adaptation over time of an adaptative therapy for a user affected by a mental disorder **(****Figure** 3), wherein the adaptative therapy is previously prescribed by a healthcare professional, the method 100 comprising:
- receiving (step 110):
   ∘ at least one user data 31 relative to the user, the at least one user data being associated with at least one symptom of the mental disorder;
   ∘ a set of C therapy categories 32, wherein each category:
      ▪ comprises a group of therapeutic exercises related to the mental disorder, and
      ▪ is associated, for each of the at least one symptom, with a predefined relation score indicative of the relatedness of the therapy category to the symptom;
- optionally, a preprocessing step similar to a function of module 12 (step 120);
- automatically adapting the therapy for the user by iteratively obtaining, for each current iteration, an adapted current therapy to be proposed to the user to improve the user current mental state by:
   ∘ obtaining the at least one user data from at least one time period anterior to the current iteration (step 130);
   ∘ ranking the therapeutic exercises based on the associated predefined relation scores and the at least one user data so as to obtain a ranked set of therapeutic exercises (step 140);
   ∘ generating the adapted current therapy 41 based on the ranked set of therapeutic exercises, so that at least one therapeutic exercise comprised in the highest ranked therapeutic exercises is included in the adapted current therapy (step 150);
- providing the adapted current therapy 41 as generated at each iteration, to be proposed to the user to improve the user current mental state (step 160).

**Figure** 2 illustrates a particular functional embodiment of the biofeedback device 2 for self-adaptation of a therapy previously prescribed by a healthcare professional to a user affected by a mental disorder. Biofeedback device 2 may be a mobile phone, a dashboard (i.e. user's dashboard), a tablet or any other suitable device.

Biofeedback device 2 is adapted to produce a user health score 42 to provide to the user a biofeedback on improvement of their current mental state.

Biofeedback device 2 comprises at least one input configured to receive (module 21):
- an adapted current therapy 41 obtained by device 1 according to any of the herein disclosed embodiments;
- at least one previous user data 33 relative to the user, the at least one previous user data 33 being associated with at least one symptom of the mental disorder previous to the prescription of the adapted current therapy 41.

The at least one previous user data 33 may be (similarly to the at least one user data 31) a health score obtained from a questionnaire such as mood-related questionnaires (e.g. PHQ-9, MADRS, HAD), a biometric data obtained from a sensor associated to the user, a temporal variation of a severity of at least one symptom related to the mental disorder. It may be collected from the user and associated at least one symptom of the mental disorder that was/were present before the user was prescribed/administered the adapted current therapy. The at least one previous user data 33 may be advantageous for evaluating the user's health status before the adapted current therapy has been provided to the user.

The adapted current therapy 41 and the at least one previous user data 33 may for example be stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

Biofeedback device 2 further comprises optionally a module 22 similar in function to module 12 of device 1.

Biofeedback device 2 may further comprise a module 23 for providing the adapted current therapy 41 to the user. In other words, module 23 may provide, administer, or present the adapted current therapy 41 to the user. In an example, module 23 delivers the adapted current therapy 41 to the user by presenting the ranked therapeutic exercises and making sure the user performs at least one of these exercises.

Biofeedback device 2 may further comprise a module 24 for collecting at least one current user data from the user. The at least one current user data may be associated with at least one symptom of the mental disorder after the user had performed at least one of the therapeutic exercises comprised in the adapted current therapy 41. Collecting at least one current user data allows for assessing the user's health status after performing a therapeutic exercise, helping to determine if the therapeutic exercise was effective in treating the symptoms of the mental disorder.

Biofeedback device 2 may further comprise a module 25 for comparing the at least one current user data to the at least previous user data 33 so as to obtain a user health score 42. The at least one current user data and the at least one previous user data may be processed (standardization, normalization, etc.) if needed. The at least one previous user data may serve as a reference for evaluating the evolution of the user's health status, whereas the current user data may is collected after performing at least one therapeutic exercise and may serve as an evaluation of the effect of the at least one therapeutic exercise on a symptom of the mental disorder. The user health score is indicative of the result of the comparison between the at least one current user data and the at least previous user data 33. In other words, it quantifies the user's symptom(s) improvement or deterioration in response to the therapeutic exercise(s). The user health score may be a binary value (e.g. 0 for deterioration and 1 for improvement), a percentage of improvement (e.g. 100% for complete recovery), a probability value, a text description (e.g. "Significant improvement", "Moderate improvement", "No change", "Deterioration").

Biofeedback device 2 may further comprise a module 26 for comparing the user health score to a predefined user health threshold. The predefined user health threshold may be defined by a healthcare professional, based on clinical evidence, or a personalized reference for the user from a time when they were not suffering from the mental disorder.

Biofeedback device 2 may further comprise a module 27 for outputting to the user the user health score 42 to provide a biofeedback on improvement of their current mental state. The user health score 42 may be presented to the user as a numerical value or a visual representation (e.g. graph, heatmap, a curve). In an embodiment, module 27 may output the user health score 42 and other user health scores obtained before the user health score 42. Advantageously, this enables the user to visualize the evolution of their health status with time.

Biofeedback device 2 may further comprise a module 28 for repeating the steps of modules 24, 25 and 26 until the user health score 42 reaches or exceeds the predefined user health threshold associated to a user health status, which means for example that the user's health status has reached or exceeded a predefined threshold of improvement of the symptom(s). The predefined threshold of improvement may be defined by a healthcare professional.

It may be observed that the operations by the modules 21, 22, 23, 24, 25, 26, 27 and 28 are not necessarily successive in time, and may overlap, proceed in parallel or alternate, in any appropriate manner.

Biofeedback device 2 may be interacting with a user interface 50, via which information can be entered and retrieved by the user. The user interface 50 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

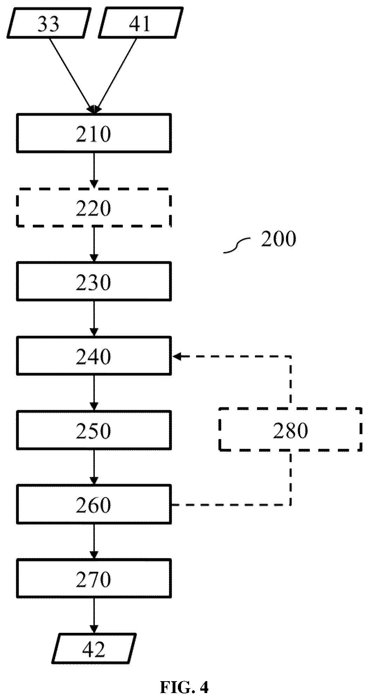

In its automatic actions, the biofeedback device 2 may for example execute the following computer-implemented method 200 for self-adaptation of a therapy previously prescribed by a healthcare professional to a user affected by a mental disorder (Figure 4), the method 200 comprising:
- receiving (step 210):
   ∘ an adapted current therapy 41 obtained by a method 100 / device 1 for automatic adaptation over time of an adaptative therapy according to any embodiment of the present disclosure;
   ∘ at least one previous user data 33 relative to the user, the at least one previous user data being associated with at least one symptom of the mental disorder previous to the prescription of the adapted current therapy;
- optionally, a preprocessing step similar to a function of module 22 (step 220);
- providing the adapted current therapy 41 to the user (step 230);
- collecting at least one current user data from said user, said at least one current user data being associated with at least one symptom of said mental disorder after the user had performed at least one of the therapeutic exercises comprised in the adapted current therapy (step 240);
- comparing said at least one current user data to said at least previous user data so as to obtain a user health score (42) (step 250);
- comparing said user health score to a predefined user health threshold (step 260);
- outputting to the user said user health score to provide a biofeedback on improvement of their current mental state (step 270);
- repeating: collect at least one current user data from said user (step 240), compare said at least one current user data (step 250), and compare said user health score (step 260), until the user health score 42 reaches or exceeds the predefined user health threshold associated to a user health status (step 280).

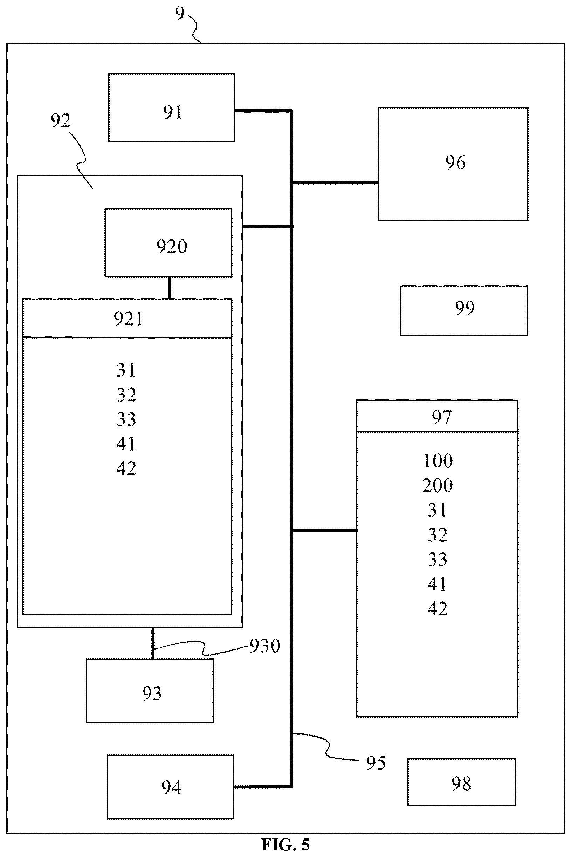

A particular apparatus 9, visible on Figure 5, is embodying the device 1 as well as the biofeedback device 2 described above. It corresponds for example to a workstation, a laptop, a tablet, a smartphone, or a head-mounted display (HMD).

That apparatus 9 comprises the following elements, connected to each other by a bus 95 of addresses and data that also transports a clock signal:
- a microprocessor 91 (or CPU);
- a graphics card 92 comprising a Graphical Processing Unit (or GPU) 920 and a Graphical Random Access Memory (GRAM) 921;
- a non-volatile memory of ROM or EPROM or EEPROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

In variant modes, the apparatus 9 may include only the functionalities of the device 1, and not the functionalities of the biofeedback device 2. In addition, the device 1 and/or the biofeedback device 2 may be implemented differently than a standalone software, and an apparatus or set of apparatus comprising only parts of the apparatus 9 may be exploited through an API call or via a cloud interface.

### EXAMPLES

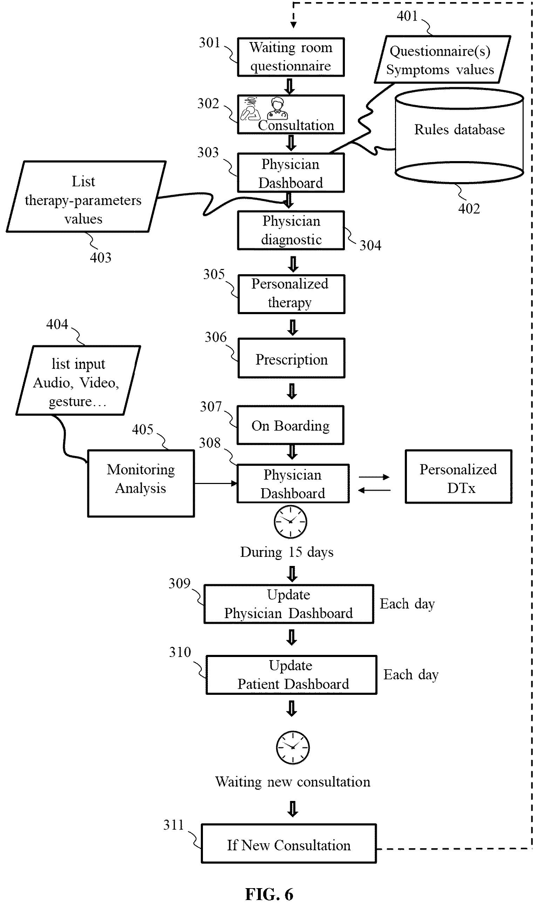

### Example 1: Patient follow-up journey (Figure 6)

Following personal problems, a user (e.g. patient) experiences a significant drop in morale and decides to consult a healthcare professional (e.g. doctor).

Step 1 (T0) (step 301), the first consultation takes place. In the waiting room, the patient completes a PHQ-9 questionnaire (dedicated to measuring the severity of depression) on a tablet provided. He obtains a score of 12 out of 36, which means that he suffers from a moderate depressive disorder.

Step 2 (T0) (step 302): The doctor scans the answers to the questionnaire in the "Doctor" dashboard (i.e. medical dashboard) and goes to pick up the patient. The doctor notices that the patient has difficulty standing up and moves very slowly. The doctor greets the patient in his office, talks to him to complete his observation and suggests that the patient uses the solution (computer-implemented method 100 and/or computer-implemented method 200). The doctor writes up his observations (patient's speed of movement, general appearance, etc.) and conclusions on the medical dashboard.

Step 3 (T0) (step 303): Based on the information provided by the doctor, a rules database "Symptoms2Therapy" (402) and the results of the questionnaire (401), the dashboard proposes a CBT/DBT therapy comprising several therapy categories (i.e. list of therapy-parameters values 403) of therapeutic exercises.

Step 4 (T0) (step 304): The doctor makes his diagnosis.

Step 5 (T0) (step 305): The doctor has full control over the therapy proposed in the medical dashboard. He validates the therapy proposal in the dashboard.

Step 6 (T0) (step 306): The doctor prescribes the solution (computer-implemented method 100 and/or computer-implemented method 200) and issues an access code for the user.

Step 7 (step 307): The patient returns to the waiting room, downloads the solution and installs it on his smartphone and/or laptop.

Step 8 (step 308), for 15 days: the solution analyzes the patient's behavior through monitoring analysis (405) by collecting for example audio, video and/or gestures input(s) from the patient (404) (e.g. through reception of at least one user data 31 and/or at least one biofeedback of the user, or though reception of at least one previous user data 33 relative to the user, through collecting 24 at least one current user data from the user). On the fifteenth day, the solution (personalized DTX) is able to make new therapeutic proposals to the user/doctor.

Step 9 (step 309): Every 24 hours, the doctor's dashboard is updated with new monitoring data. The patient's attitude to DTx is assessed: time spent on exercises and completion of each exercise are measured. The solution fine-tunes the therapy and informs the doctor via the medical dashboard. The solution explains the results of its analysis and the reason for programming certain categories of exercise.

Step 10 (step 310): Every 24 hours, the patient's dashboard is updated with new monitoring data.

Step 11 (step 311): If the doctor decides to have a second consultation, it is returned to Step 1.

### Example 2: Optimizing the digital therapy

It is proposed two ways of implementing the digital therapy. The first (illustrated in **figure 7****)** consists of a simple evaluation of the priority of exercise categories, while the second proposes a more refined mechanism that takes into account symptom severity, physician (healthcare professional) and patient (user) choices, and the effectiveness of each exercise.

A priori information: Exercise categories have a variable effect on different symptoms. The aim is to reduce symptoms by implementing the most appropriate exercise categories. There is therefore a link between symptoms and exercise category: the stronger the link, the greater the effect of an exercise category on a given symptom.

It is postulated that it has first assessed the value of the link between each category and each symptom. This link was evaluated after interviewing some 50 psychiatrists. The average value of this link was therefore calculated after the interviews. These are the default link values proposed in the system. The attending physician can specify the link values they prefer.

### Example 3: Optimizing the digital therapy

Each therapy category is associated with a priority score. The aim is to be able to classify the therapy categories according to their priority score and present the patient with the therapy categories with the highest priority scores. At the start, all therapeutic exercises within the same therapy category may have the same priority score. However, as adaptation progresses, the priority score of an individual therapeutic exercise can change and become different from priority scores of other therapeutic exercises.

Digital therapy (DTx) personalization consists in specifying the therapy categories that minimize the severity of the patient's symptoms. As an example, let's consider a patient responding to the questionnaire and presenting two symptoms: S6 and S2. **Figure 7** illustrates the point:

Symptom S6 is associated with question 6 of the PHQ-9 (Feeling bad about yourself or that you are a failure or have left yourself or your family done). This symptom could be strongly reduced (strongly is characterized by W_{C1,S6} = 0.9 see figure 7) if the patient followed the exercises belonging to category C1 "self-confidence".

The same symptom could perceptibly decrease (perceptibly is characterized by W_{C3,S6} = 0.4) if the patient followed the exercises belonging to category C3 "Dynamism".

Category C2 corresponds to "Social relations".

The patient's second symptom S2 is associated with the second question of the questionnaire (Feeling down, depressed, or hopeless). Exercises in category C1 were likely to influence symptom S2, with a rather weak effect of W_{C1,S2} = 0.3.

It is proposed to calculate a priority score for each Cᵢ category that takes into account the a priori evaluated effect of each category of therapeutic exercises on each symptom. Let S be the set of symptoms and a subset S_{c} ⊆ S, S_{c} is the subset of symptoms on which the Cᵢ category acts. Let P_{Ci} be the priority score of category Cᵢ. For each category Cᵢ : P_{Ci}= Σ_{k∈Sc} W_{C,k} * SEₖ , with the index k running over the set of symptoms having an effect on the category considered Cᵢ; SEₖ being the severity of symptom number k ( $k ∈ ℕ *$).

In Figure 7 the following values are used: The severity of symptoms 2 and 6, automatically evaluated in the monitoring module: SE2 = 0.3; SE6 = 0.6. (SE1 = 0, SE3 = 0, SE4 = 0, SE5 = 0, SE7 = 0, SE8 = 0, SE9 = 0)

Measurement of the effect of therapy categories on symptoms, values evaluated a priori: W_{C1,S2} = 0.3, W_{C1,S6} = 0.9, W_{C3,S6} = 0.4.

Category priority scores are as follows : P_{C1} = SE2*W_{C1,S2} + SE6*W_{C1,S6} = 0.2*0.3+ 0.6*0.9 = 0.6; P_{C2} = 0.0; P_{C3} = SE6*W_{C3,S6} = 0.6*0.4 = 0.24; this means that exercises relating to category C₁ will be completed before those relating to category C₃. W_{C1,S2} corresponds to the relation score of therapy category 1 (C1) to symptom S2. W_{C1,S6} corresponds to the relation score of therapy category 1 (C1) to symptom S6. W_{C3,S6} corresponds to the relation score of therapy category 3 (C3) to symptom S6.

Indeed, **figure 7** shows that it is sufficient to know in advance the values of the predefined relation scores and the severity of each of the symptoms to be able to establish a priority score for each of the therapeutic exercises. (the severity of the symptoms is specified in data 31 of module 11 in **figure 1****).**

### Example 4: Fine-tuning at exercise level

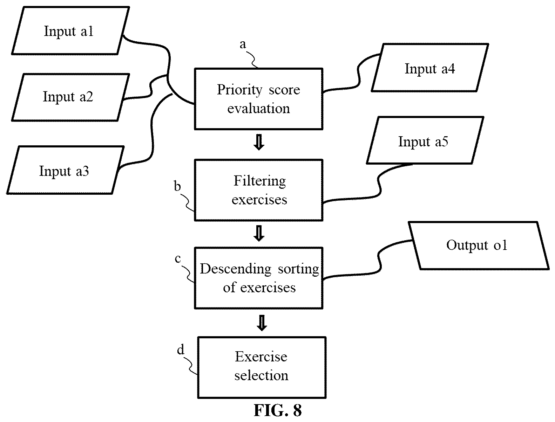

Following on from optimizing the digital therapy (cf. example 3), a second implementation is proposed that considers both physician choice and exercise effectiveness when calculating priority scores. Dynamic symptom minimization takes place in several stages, as illustrated in **figure 8****,** the steps of which are described below.

Step a: calculate/evaluate a priority score for each exercise using the following input parameters:
- Symptom(s) severity (input a1) calculated in the "Monitoring Analysis" step;
- The (time) derivative of symptom(s) severity (input a2);
- The history of exercises already performed in the past (input a3) and their impact on the positive or negative evolution of symptoms;
- Doctor's preferences (input a4): The doctor can select from his dashboard the N symptoms he feels are most relevant to prioritize;
- Patient preferences (input a5): The patient can choose the exercises that feel good. They can also choose the exercises they don't like. It's important to collect the patient's preferences to maximize adherence to digital therapy. This adherence favors taking the medication prescribed by the doctor and maximizes his chances of reducing symptoms. Each of these input parameters is associated with a weighting coefficient chosen to reflect the relative importance of the different input criteria.

Step b: Patient preferences (input a5) include, among other things, exercises that the patient does not want to do; this information is taken into account to remove them from the exercise list. Step b comprises filtering/removal of exercises to be banned.

Step c: Sort (ranking) remaining exercises in descending order. The value of the priority score evaluated in Step a is used to order the exercises to be performed in descending order. In the event of a tie, category diversity is favored: among exercises with the same score, the one whose category is least represented among the exercises already selected is chosen. The output (output o1) of this step is the exercises list with priority scores.

Step d: (Exercise selection presented at the current moment) Once an exercise has been completed, it is removed from the descending list generated in step c. In the event of a tie, category diversity is preferred: among exercises with the same score, the one whose category is least represented among the exercises already selected is chosen first.

### Example 5:

Outline the overall process: Every week n, the system:
(1) Observes the input data: symptom severities, recent changes in severity, exercise history, patient preferences, and medical opinion, if any.
(2) Computes, for each exercise, a priority score that takes into account the evolution in symptom severity, current severity, medical priorities, past effectiveness, and patient preferences.
(3) Filters out the banned exercises and sorts the remaining exercises by priority order.
(4) Builds a "pool" of exercises to be recommended to the patient during the week.

### Notations:

- Let S = {1, 2, ... ,N_{S}} be the set of symptoms.
- Let C = {1, 2, ... ,Nc} be the set of CBT categories.
- Let E = {1, 2, ... ,N_{E}} be the set of exercises.
Each exercise Eᵢ is associated with exactly one category C(Eᵢ) ∈ C.

### Input data:

- Weekly severities: For each symptom k ∈ S, let Sₖ(n) ∈ [0, 1] be the normalized severity at the beginning of week n. A value close to 1 indicates high severity.
- Exercise success history: The past history of exercises already carried out, and their observed impact (success) on symptom severity.
- Medical opinion (optional): An ordered tuple (pi, p₂, p₃) ∈ S³ of priority symptoms for week n.
- Patient preferences: E_{pref} is the set of the patient's favorite exercises, and E_{ban} is the set of exercises to be banned.

### Parameters:

- Effect of categories on symptoms: For each category c ∈ C, it is defined a subset S_{c} ⊆ S of symptoms on which category c acts, and a weight function w_{c,k} ∈ [0, 1] for each symptom k ∈ S, representing the expected relative level of impact of exercises in category c on symptom k. S_{c} = {k ∈ S | w_{c,k} > 0}.
- Score weighting coefficients: α, β, γ, δ, ζ ≥ 0 are coefficients that modulate the relative importance of the change in symptom severity (α), the absolute symptom severity (β), medical priorities (γ), past success (δ), and patient preferences (ζ) in the computation of an exercise's priority.
- Memory parameter: ρ > 0 in the exponentially decaying function λ(tⱼ) = e^{-ρ(T-tⱼ)} for weighting the exercise history.

### Methodology:

Constructing the priority score of an exercise: the aim is to build a priority score for each exercise, which reflects the importance of recommending that exercise to the patient during a given week n. This importance should be linked to the change in symptoms as well as their absolute state, the doctor's opinion, the exercise's past success rate, and patient preferences. Below are defined the different terms intended to compose each exercise's priority score.

Symptom changes (by category): Let ΔSₖ(n) be the normalized change in severity of symptom k from the previous week to the current week. More precisely: ΔSₖ(n) = Sₖ(n) - Sₖ(n - 1). Since Sₖ( ▪ ) ∈ [0, 1], then ΔSₖ(n) ∈ [-1, 1].

### Interpretation:

- ΔSₖ(n) = 1: sharp deterioration of the symptom (significant increase in severity).
- ΔSₖ(n) = -1: sharp improvement of the symptom (significant decrease in severity).
To ensure consistency with other terms, a positive normalized version is used: ${ΔS}_{k}^{norm} \left(n\right) = \frac{{ΔS}_{k} \left(n\right) + 1}{2} ∈ \left[0, 1\right] .$ Thus:
- ${ΔS}_{k}^{norm} \left(n\right) = 1$ : major deterioration
- ${ΔS}_{k}^{norm} \left(n\right) = 0$ : major improvement
Then, for each category c, the weighted average of the severity changes ${ΔS}_{k}^{norm} \left(n\right)$ for the symptoms on which c acts is computed: $\sum ΔS \left(n, c\right) = \frac{{\sum }_{k ∈ {S}_{C}} {w}_{c , k} {ΔS}_{k}^{norm} \left(n\right)}{{\sum }_{k ∈ {S}_{C}} {w}_{c , k}}$. A high value of ΣΔS(n, c) indicates a high average deterioration, thus justifying a higher priority.

Symptom severity (by category): From the normalized severity scores Sₖ(n) for each symptom k, it is computed, for each category c, the weighted average severity of the symptoms on which c acts: $\sum S \left(n, c\right) = \frac{{\sum }_{k ∈ {S}_{C}} {w}_{c , k} {S}_{k} \left(n\right)}{{\sum }_{k ∈ {S}_{C}} {w}_{c , k}}$. A high value of ΣS(n,c) indicates a high average severity, thus justifying a higher priority.

Medical priorities: If the doctor provides a set of priority symptoms (p₁, p₂, p₃), it is defined, for each category c: ${μ}_{1} \left(c\right) = \left\{\begin{matrix}1 if {p}_{1} ∈ {S}_{c} \\ 0 otherwise\end{matrix}\right. , {μ}_{2} \left(c\right) = \left\{\begin{matrix}1 if {p}_{2} ∈ {S}_{c} \\ 0 otherwise\end{matrix}\right. , {μ}_{3} \left(c\right) = \left\{\begin{matrix}1 if {p}_{3} ∈ {S}_{c} \\ 0 otherwise\end{matrix}\right.$ Aggregation: $\sum {S}_{doctor} \left(c\right) = \frac{{μ}_{1} \left(c\right)}{2} + \frac{{μ}_{2} \left(c\right)}{3} + \frac{{μ}_{3} \left(c\right)}{6} ∈ \left[0, 1\right]$. A high value of ΣS_{doctor}(c) indicates a high medical priority for that category, thus justifying a higher recommendation priority.

Past success criterion Success_{Eᵢ}: the criterion Success_{Eᵢ} measures the past effectiveness of exercise Eᵢ.

Time definition: Let exercise Eᵢ have been performed in the past at times {t₁,..., tₘ}. Let T be the current time. It is defined an exponentially decaying weighting function: λ(tⱼ) = e^{-ρ}(^{T-tⱼ}), ρ > 0. More recent occurrences (T - tⱼ is small) will thus be more strongly weighted.

Impact measurement per occurrence: For each occurrence j: ${X}_{k}^{before} \left({t}_{j}\right) = average of {X}_{k} \left(t\right) on \left[{t}_{j}-12h{, t}_{j}\right]$ ${X}_{k}^{after} \left({t}_{j}\right) = average of {X}_{k} \left(t\right) on \left[{t}_{j},{t}_{j}+12h\right]$ where Xₖ(t) ∈ [0, 1] is the severity of symptom k at time t. The variation for each targeted symptom k ∈ S_{c} (the category of Eᵢ) is: ${D}_{{E}_{i} , k} \left({t}_{j}\right) = {X}_{k}^{before} \left({t}_{j}\right) - {X}_{k}^{after} \left({t}_{j}\right)$. If D_{Eᵢ,k}(tⱼ) > 0, it means the exercise helped improve the symptom (post-exercise severity decreased). If D_{Eᵢ,k}(tⱼ) < 0, it means the exercise may have worsened the symptom (post-exercise severity increased). Then the weighted average over S_{c} is computed: D_{Eᵢ}(tⱼ) = $\frac{{\sum }_{k ∈ {S}_{c}} {w}_{c , k} {D}_{{E}_{i} , k} \left({t}_{j}\right)}{{\sum }_{k ∈ {S}_{c}} {w}_{c , k}} ∈ \left[-1,1\right]$.

Normalization of the success criterion: ${D}_{{E}_{i}}^{norm} \left({t}_{j}\right) = \frac{{D}_{{E}_{i}} \left({t}_{j}\right) + 1}{2} ∈ \left[0, 1\right]$.

Historical aggregation: if exercise Eᵢ has been performed at least once in the past (m > 0): ${Success}_{{E}_{i}} = \frac{{\sum }_{j = 1}^{m} λ \left({t}_{j}\right) {D}_{{E}_{i}}^{norm} \left({t}_{j}\right)}{{\sum }_{j = 1}^{m} λ \left({t}_{j}\right)}$. If there is no history for Eᵢ (m = 0), then by default: Success_{Eᵢ}= 0.5.

Patient preferences: a preference indicator is defined for each exercise: ${Pref}_{{E}_{i}} = \left\{1 if {E}_{i}∈\right. {E}_{pref} \left(\begin{matrix}the exercise is one he patient ' s favorites \\ 0 otherwise\end{matrix}\right)$

Aggregation of terms: for an exercise Eᵢ ∈ E, belonging to category c = C(Eᵢ), its weekly priority score is defined: P_{Eᵢ}(n) = α ΣΔS(n, c) + β ΣS(n, c) + γ ΣS_{doctor} (c) + δ Success_{Eᵢ}+ ζ Pref_{Eᵢ}. Coefficients α, β, γ, δ, ζ ≥ 0 are chosen to reflect the relative importance of the different criteria. α > β to give more weight to symptom changes ( ${ΔS}_{k}^{norm} \left(n\right)$) than to their absolute values (Sₖ(n)). γ = 0 if no medical opinion is available, otherwise γ ≠ 0. δ is chosen sufficiently large to strongly favor exercises that have demonstrated good effectiveness in the past.

### Exercise selection for recommendation :

Filtering and sorting :
1. Remove from E all exercises in E_{ban}.
2. Compute P_{Eᵢ}(n) for each Eᵢ ∈ E\E_{ban}.
3. Sort the remaining exercises in decreasing order of P_{Eᵢ}(n).
4. In case of a tie, category diversity is favored: among equally scored exercises, the one whose category is least represented among the already selected exercises is selected first.

Pool selection and recommendation: let k be the number of exercises to retain. After filtering and sorting, it is defined: ${E}_{n}^{pool} = \left\{{E}_{i 1},{E}_{i 2},…,{E}_{ik}\right\}$, where (Eᵢ₁, ..., Eᵢₖ) are the k top exercises by score P_{Eᵢ}(n). Whenever a recommendation is made, an exercise is picked in order from the list ${E}_{n}^{pool}$.

## Claims

1. A device (1) for automatic adaptation over time of an adaptative therapy for a user affected by a mental disorder, wherein said adaptative therapy is previously prescribed by a healthcare professional, said device (1) comprising:
- at least one input configured to receive (11):
∘ at least one user data (31) relative to the user, said at least one user data being associated with at least one symptom of said mental disorder;
∘ a set of C therapy categories (32), wherein each category:
▪ comprises a group of therapeutic exercises related to said mental disorder, and
▪ is associated, for each of said at least one symptom, with a predefined relation score indicative of the relatedness of said therapy category to said symptom;
- at least one processor configured to automatically adapt said adaptative therapy for the user by iteratively obtaining, for each current iteration, an adapted current therapy to be proposed to the user to improve the user current mental state by:
∘ obtaining (13) the at least one user data from at least one time period anterior to the current iteration;
∘ ranking (14) said therapeutic exercises based on said associated predefined relation scores and said at least one user data so as to obtain a ranked set of therapeutic exercises;
∘ generating (15) the adapted current therapy (41) based on said ranked set of therapeutic exercises, so that at least one therapeutic exercise comprised in the highest ranked therapeutic exercises is included in the adapted current therapy;
- at least one output configured to provide (16) said adapted current therapy (41), as generated at each iteration, to be proposed to the user to improve the user current mental state.

2. The device (1) according to claim **1,** being further configured to receive as input:
- a history of the therapeutic exercises, comprising a collection of therapeutic exercises previously performed by the user and an associated biofeedback for each therapeutic exercise previously performed;
- at least one biofeedback of the user, each of said at least one biofeedback relating to the effect of one therapeutic exercise;
wherein said at least one processor is further configured to:
- update the history of the therapeutic exercises using the latest at least one biofeedback of the user;
- for each therapeutic exercise of each therapy category, estimate a user exercise weight being representative of an effect of said therapeutic exercise using said updated history of the therapeutic exercises,
- obtain a priority score for each therapeutic exercise in each therapy category using said at least one user exercise weight, said at least one user data and said predefined relation scores; and
wherein the ranking (14) of the therapeutic exercises is performed using said priority scores and wherein the at least n higher ranked therapeutic exercises are added to the adapted current therapy.

3. The device (1) according to any one of claims **1** to **2,** wherein said at least one user data relative to the user is one of the following:
- a health score obtained from a questionnaire;
- a biometric data obtained from a sensor associated to the user;
- a temporal variation of a severity of at least one symptom related to said mental disorder.

4. The device (1) according to any one of claims **1** to **3,** wherein each category of said set of C therapy categories is one of the following:
- learning a behavior, such as assertiveness;
- learning awareness of maladaptive and unrealistic thoughts;
- learning to accept various emotions;
- learning to relax physically and psychologically;
- gradual exposure to a stressful situation, such as phobia;
- increasingly frequent exposure to rewarding situations;
- mindfulness exercises;
- Progressive Muscle Relaxation (PMR) exercices;
- developing human relations through role-playing or a game;
- Dialectical Behavior Therapy (DBT) exercises;
- Acceptance and Commitment Therapy (ACT) exercises;
- Cognitive Behavioral Therapy (CBT) exercises;
- Rational Emotive Behavior Therapy (REBT) exercises;
- Positivist Psychology exercices.

5. The device (1) according to any one of claims **1** to **4,** being further configured to receive as input a preference of said user, and wherein said ranking of said therapeutic exercises is further based on said preference of said user.

6. The device (1) according to any one of claims **1** to **5,** wherein, before ranking said therapeutic exercises, said set of C therapy categories is filtered according to a received preference of said healthcare professional.

7. The device (1) according to any one of claims **1** to **6,** wherein said at least one user data is obtained according to a predefined schedule from the user so as to update said current therapy on said predefined time interval.

8. The device (1) according to any one of claims **1** to **7,** wherein said predefined relation score is reported from a relation data structure comprising predefined relation scores for each symptom associated to said mental disorder and each therapy category, so as to associated a predefined relation score to a pair symptom - therapy category.

9. The device (1) according to any one of claims **1** to **8,** wherein information relating to said adapted current therapy is provided to the user through an interface, and said ranked therapeutic exercises are presented to the user in a descending rank order by: presenting first a therapeutic exercise with the highest rank and last a therapeutic exercise with the lowest rank.

10. The device (1) according to any one of claims **1** to **9,** wherein, when two therapeutic exercises have an equal rank, the therapeutic exercise associated with the longest time interval since its previous use is presented before the therapeutic exercise associated with a shorter time interval since its previous use.

11. The device (1) according to any one of claims **1** to **10,** wherein in the current therapy under administration is updated by removing a therapeutic exercise once said user uses said therapeutic exercise.

12. The device (1) according to any one of claims **1** to **11,** wherein a predefined time interval elapses between two consecutive iterations.

13. A biofeedback device (2) for self-adaptation of an adaptative therapy previously prescribed by a healthcare professional to a user affected by a mental disorder, said biofeedback device (2) comprising:
- at least one input configured to receive (21):
∘ an adapted current therapy (41) obtained by a device (1) for automatic adaptation over time of an adaptative therapy for a user affected by a mental disorder according to any one of claims **1** to **12;**
∘ at least one previous user data (33) relative to the user, said at least one previous user data (33) being associated with at least one symptom of said mental disorder previous to the prescription of said adapted current therapy (41);
- at least one processor configured to:
∘ provide (23) said adapted current therapy (41) to said user;
∘ collect (24) at least one current user data from said user, said at least one current user data being associated with at least one symptom of said mental disorder after the user had performed at least one of the therapeutic exercises comprised in the adapted current therapy (41);
∘ compare (25) said at least one current user data to said at least previous user data (33) so as to obtain a user health score (42);
∘ compare (26) said user health score to a predefined user health threshold;
∘ output (27) to the user said user health score (42) to provide a biofeedback on improvement of their current mental state;
∘ repeat (28): collect (24) at least one current user data from said user, compare (25) said at least one current user data, and compare (26) said user health score, until the user health score (42) reaches or exceeds the predefined user health threshold associated to a user health status.

14. A computer-implemented method (100) for automatic adaptation over time of an adaptative therapy for a user affected by a mental disorder, wherein said adaptative therapy is previously prescribed by a healthcare professional, said method (100) comprising:
- receiving (110):
∘ at least one user data (31) relative to the user, said at least one user data being associated with at least one symptom of said mental disorder;
∘ a set of C therapy categories (32), wherein each category:
▪ comprises a group of therapeutic exercises related to said mental disorder, and
▪ is associated, for each of said at least one symptom, with a predefined relation score indicative of the relatedness of said therapy category to said symptom;
- automatically adapting said adaptative therapy for the user by iteratively obtaining, for each current iteration, an adapted current therapy to be proposed to the user to improve the user current mental state by:
∘ obtaining (130) the at least one user data from at least one time period anterior to the current iteration;
∘ ranking (140) said therapeutic exercises based on said associated predefined relation scores and said at least one user data so as to obtain a ranked set of therapeutic exercises;
∘ generating (150) the adapted current therapy (41) based on said ranked set of therapeutic exercises, so that at least one therapeutic exercise comprised in the highest ranked therapeutic exercises is included in the adapted current therapy;
- providing (160) said adapted current therapy (41) as generated at each iteration, to be proposed to the user to improve the user current mental state.

15. A computer-implemented method (200) for self-adaptation of an adaptative therapy previously prescribed by a healthcare professional to a user affected by a mental disorder, said method (200) comprising:
- receiving (210):
∘ an adapted current therapy (41) obtained by a method (100) for automatic adaptation over time of an adaptative therapy according to claim **14;**
∘ at least one previous user data (33) relative to the user, said at least one previous user data being associated with at least one symptom of said mental disorder previous to the prescription of said adapted current therapy;
- providing (230) said adapted current therapy (41) to said user;
- collecting (240) at least one current user data from said user, said at least one current user data being associated with at least one symptom of said mental disorder after the user had performed at least one of the therapeutic exercises comprised in the adapted current therapy;
- comparing (250) said at least one current user data to said at least previous user data so as to obtain a user health score (42);
- comparing (260) said user health score to a predefined user health threshold;
- outputting (270) to the user said user health score to provide a biofeedback on improvement of their current mental state;
- repeating (280): collect at least one current user data from said user (240), compare said at least one current user data (250), and compare said user health score (260), until the user health score (42) reaches or exceeds the predefined user health threshold associated to a user health status.
